# EUROPÄISCHE PATENTSCHRIFT

(11) **EP 4 200 009 B1**
(45) Veröffentlichungstag und Bekanntmachung des Hinweises auf die Patenterteilung: **17.07.2024**
(21) Anmeldenummer: 21772830.2
(22) Anmeldetag: 18.08.2021
(51) Int. Cl.: A61N 1/36, A61N 1/32

(54) **VORRICHTUNG ZUR STIMULATION DES HAARWUCHSES**
DEVICE FOR STIMULATING HAIR GROWTH
DISPOSITIF POUR STIMULER LA POUSSE DES CHEVEUX

(30) Priorität: 19.08.2020 DE 102020121715
(43) Veröffentlichungstag der Anmeldung: 28.06.2023
(73) Patentinhaber: Mane Biotech GmbH, 50858 Köln (DE)
(72) Erfinder: JELLARD, Samuel Christopher Jack, 50858 Köln (DE); CHACÓN MARTÍNEZ, Carlos Andrés, 50858 Köln (DE)
(74) Vertreter: Castell, Klaus
(86) Internationale Anmeldenummer: PCT/IB2021/057585
(87) Internationale Veröffentlichungsnummer: WO 2022/038529

(56) Entgegenhaltungen:
- US-A1- 2010 217 369
- US-A1- 2018 326 208
- US-A1- 2019 143 138

## Beschreibung

Die Erfindung betrifft eine Vorrichtung zur Stimulation des Haarwuchses, aufweisend mindestens eine Elektrode zur elektrischen Reizung der Haut, wobei eine mit der mindestens einen Elektrode verbundene Steuerungsvorrichtung elektrische Impulse auslöst.

Es ist bekannt, dass die elektrische Stimulation der Kopfhaut bei einsetzendem Haarverlust die Haarfollikel zu neuem Haarwachstum anregen kann. Zur Stimulation der Kopfhaut ist es notwendig, Elektroden auf die Kopfhaut zu setzen. Durch die Elektroden fließt elektrischer Strom, der über die Elektroden in die Kopfhaut fließt. Für einen therapeutischen Ansatz hat sich diese Methode bisher nicht geeignet, da die elektrische Stimulation zum Teil mehrere Stunden pro Tag stattfinden muss, um den erwünschten Effekt zu erzielen.

In dem koreanischen Patent KR 100 671 943 B1 wird eine Haube offenbart, in der Elektroden vorhanden sind, um den Haarwuchs zu stimulieren. Diese Haube wird ähnlich wie eine Badehaube getragen und eignet sich für nicht für den täglichen Einsatz einer zum Beispiel im Berufsleben befindlichen Person.

In der US-Patentanmeldung US 2010/0217369 A1 wird eine helmartige Vorrichtung gelehrt, in welcher eine Kontrollvorrichtung und Elektroden zur Kopfhautstimulation vorhanden sind.

In der US-Patentanmeldung US 2019/143138 A1 wird eine kappenartige und eine föhnhaubenartige Vorrichtung zur Stimulation des Haarwuchses offenbart, welche über eine optische Stimulation, also mit einer Lichttherapie, den Haarwuchs stimuliert.

In der US-Patentanmeldung US 2018-0326208 A1 wird eine Vorrichtung zur elektrischen Stimulation des Haarwuchses offenbart, welches über ein Nadelrad, das über die Kopfhaut gerollt wird, die elektrische Stimulation ausübt. Das Nadelrad sticht dabei mit sehr kleinen Nadeln in die obere Epidermis ein und erzeugt mei der Stimulation eine Wundelektrolyse.

Schließlich wird in der internationalen Patentanmeldung WO 01/02051 A1 eine Magnetanordnung im Inneren einer Baseball-Kappe gelehrt, die den Haarwuchs stimulieren soll.

Alle zuvor genannten Vorrichtungen haben den Nachteil, dass die elektrische Stimulation an den tatsächlichen Haarverlust angepasst werden muss. Bei einem kreisförmigen Haarausfall sollte die Stimulation bevorzugt nur an den Stellen des Haarverlustes stattfinden. Es ist also eine stetige Anpassung der Elektroden notwendig. Haarwuchsstimulationsvorrichtungen in Form einer Haube oder in Form eines Helms können daher nur ein einer einzigen Position getragen werden. Bei längeren Tragezeiten der Stimulationsvorrichtung verringert sich die sogenannte Compliance, also die Bereitschaft der Person, die Vorrichtung in der vordefinierten Stellung auf dem Kopf zu tragen oder wegen Druckstellen nicht tragen zu können. Es ist auch möglich, dass der Haarverlust auch und gerade in den Übergangsbereichen von Bewuchs zu ausgedünnt bis kahl stimuliert wird. Um diese Bereiche gezielt zu stimulieren, ist es notwendig, dass die Stimulationsvorrichtung diese Bereiche auch selbsttätig findet.

Um die elektrische Stimulation der Haarfollikel zu erreichen, wird im Stand der Technik vorgeschlagen, dass die elektrischen Impulse in die Haut über nadelförmige Elektroden eingebracht werden. Die dafür eingesetzten Nadeln sind sehr dünn, etwa wie Akkupunkturnadeln, und schmerzen daher kaum, wenn sie in den oberen Hautschichten stecken. Das Einstechen der Elektroden erfordert jedoch eine hohe Überwindung über den möglichen Schmerz beim Applizieren. In der Haut steckende nadeln oder Elektroden verringern die Compliance noch weiter. Die in der Hau steckenden nadeln sind des Weiteren ortsfest. Eine fortwährende Anpassung der elektrischen Stimulation ist nicht möglich, etwa wenn die Vorrichtung zur Stimulation der Haut über den Tag ähnlich wie eine Kappe vom Nutzer auf dem Kopf verrutscht wird.

Aufgabe der Erfindung ist es daher, eine Vorrichtung zur Stimulation des Haarwuchses mit erhöhtem Tragekomfort zur Verfügung zu stellen, welche außerdem selbsttätig die Stimulation an den lokalen Haarstatus anpasst.

Die erfindungsgemäße Aufgabe wird gelöst durch eine Vorrichtung zur Stimulation des Haarwuchses mit den Merkmalen nach Anspruch 1. Weitere vorteilhafte Ausgestaltungen der erfindungsgemäßen Vorrichtung zur Stimulation des Haarwuchses sind in den Unteransprüchen zu Anspruch 1 angegeben.

Nach dem Gedanken der Erfindung ist also vorgesehen, eine Steuerungs- und Detektionsvorrichtung vorzusehen, welche stets den tatsächlichen Haarverluststatus oder die tatsächliche Behaarung messen und detektieren kann. Die Steuerungsvorrichtung führt in Abhängigkeit des detektierten Haarverluststatus oder der detektierten Behaarung ein Stimulationsprogramm durch. Die Steuerungs- und Detektionsvorrichtung kann dabei Temperatursensoren beinhalten, Positionierungssensoren, um die Position der Vorrichtung auf der Kopfhaut zu detektieren, Kontakt- und/oder Näherungssensoren, um den adäquaten Kontakt der Vorrichtung mit der Haut zu detektieren, Leitfähigkeitssensoren, um Änderung der Leitfähigkeit der Kopfhaut zu detektieren und die Therapie darauf hin entsprechend anzupassen. Detektiert die Vorrichtung beispielsweise den Verlust von Haaren in den sogenannten Geheimratsecken, so werden Elektroden bevorzugt in dieser Region mit elektrischen Stimulationspulsen beaufschlagt. Der besondere Vorteil der steten Messung des Haarverluststatus oder der Beharrung besteht besonders darin, dass sich die Vorrichtung an die aktuelle Position der Stimulationsvorrichtung auf dem Kopf oder an der betreffenden Körperstelle anpasst. Es ist somit möglich, die Vorrichtung zu verrücken, wenn der Tragekomfort, beispielsweise durch ein empfundenes Druckgefühl oder durch ein Jucken der Kopfhaut oder der Haut an den Tragestellen, reflektorisch mit einem Umsetzen der Vorrichtung vom Träger und Nutzer beantwortet wird.

Für eine verlässliche Detektion können Folienelektroden eingesetzt werden, die eine Flächendetektion ermöglichen. Es ist aber auch möglich, dass vorgesehen ist, dass die Steuerungsvorrichtung mit einer Mehrzahl von Detektorvorrichtungen verbunden ist, wobei jede einzelne Detektorvorrichtung einen vorbestimmten Bereich der Kopfhaut oder der betreffenden Haut abdeckt. Dabei kann die Detektorvorrichtung den jeweils lokalen Haarverluststatus oder die lokale Behaarung über eine Impedanzmessung detektieren. Da das Haar einerseits elektrisch isolierend wirkt und auch eine Elektrode von der Haut beabstandet, wirkt das Haar wie ein Dielektrikum zwischen der elektrisch Leitfähigen Haut und der Elektrode.

Zur Übertragung der elektrischen Reize können Spikes auf der Elektrode vorhanden sein, die eine runde Spitze aufweisen und durch noch vorhandenes Haar ragen, jedoch auf der Kopfhaut aufliegen und die Haut nicht durchdringen. Es handelt sich bei den runden Spikes auf der Oberfläche um nichtinvasive Elektroden.

Feuchtigkeit und Schweiß können die Leitfähigkeit der Haut stark beeinflussen. Es ist daher vorgesehen, dass die Steuerungsvorrichtung des Weiteren mit einem Feuchtigkeitssensor verbunden ist, über welche die elektrische Leitfähigkeit der Haut ableitbar ist, wobei die Steuerungsvorrichtung in Abhängigkeit von der detektierten Feuchtigkeit das voreingestelltes Stimulationsprogramm variiert. Es ist so möglich, bei Detektion eines schweißfeuchten Kopfes oder schweißfeuchten Haut die Höhe der elektrischen Spannung zur Stimulation der Haut zu verringern, um dem Nutzer nicht mit unangenehmen bis schmerhaften elektrischen Impulsen zu stören.

In noch weiterer und vorteilhafter Ausgestaltung der erfindungsgemäßen Vorrichtung zur Stimulation des Haarwuchses kann vorgesehen sein, dass die Steuerungsvorrichtung des Weiteren mit einer Vorrichtung zur drahtlosen Datenübertragung verbunden ist, wie beispielsweise ein BlueTooth^{®}-Modul oder ein WLAN-Modul, über welche der Status der aktuelle Steuerungsvorrichtung auslesebar ist. Es ist somit möglich, die Steuerungsvorrichtung intelligent zu machen, in dem das Stimulationsprogramm auf die Tragegewohnheiten des Nutzers reagiert. Die Intelligenz kann aus einem Programm in einem entfernten Computer stammen, aus einem Mobiltelefon mit ausreichender Rechenleistung. Des Weiteren kann die Datenfernübertragung zu einem behandelnden Arzt geschehen, der die Therapie zum Haarwuchs kontrolliert und überwacht. Es ist nicht nur möglich, dass die Daten aus der erfindungsgemäßen Vorrichtung zur Stimulation des Haarwuchses nach außen abgegeben werden, sondern es ist auch möglich, dass das Verhalten die Steuerungsvorrichtung von außen kontrollierbar ist, in dem Datenfluss von außen an die Steuerungsvorrichtung gerichtet ist. Es kann also vorgesehen sein, dass die Steuerungsvorrichtung des Weiteren mit einer Vorrichtung zur drahtlosen Übertragung verbunden ist, wie beispielsweise ein BlueTooth^{®}-Modul oder ein WLAN-Modul, über welche der Status der Steuerungsvorrichtung veränderbar ist. Eine Änderung des Status kann sein, dass die aktivierten Elektroden angepasst werden, dass die Parameter zur Erkennung der stimulationswürdigen Bereiche von außen angepasst werden oder auch, dass ein vordefiniertes Programm zur Stimulation angepasst wird.

In besonders vorteilhafter Ausgestaltung der Vorrichtung kann vorgesehen sein, dass die Vorrichtung in Form eines Hutes oder einer Kappe aufgebaut ist, wobei die mindestens eine Elektrode und die mindestens eine Detektorvorrichtung innerhalb des Hutes oder innerhalb der Kappe angeordnet sind. Dabei bietet sich als Ort der Elektrode und als Ort der mindestens einem Detektorvorrichtung das Hutfutter oder das Kappenfutter an.

Für eine besonders unproblematischen und vielseitigen Einsatz hat es sich als vorteilhaft erwiesen, wenn vorgesehen ist, dass innerhalb des Hutes oder innerhalb der Kappe ein Akkumulator zur Stromversorgung angeordnet ist. Dadurch wird der Träger unabhängig von einer Stromversorgung und auch von einem Kabel, dass gegebenenfalls zu einer Stromquelle führt, die am Körper getragen wird. Um den Akkumulator zu laden, kann vorgesehen sein, dass der Hut oder die Kappe zum Laden des Akkumulators ein Stromversorgungskabel aufweist, oder dass im Hut oder in der Kappe eine Spule zur induktiven Ladung des Akkumulators vorhanden ist. Die Vorrichtung zur Stimulation des Haarwuchses in Form eines Hutes oder in Form einer Kappe kann dann auf einer Aufbewahrungsvorrichtung, ähnlich einem Perückenständer, induktiv aufgeladen werden.

Zur Stimulation des Haarwuchses kann vorgesehen sein, dass die die Steuerungsvorrichtung die Reizung der Haut mit folgenden elektrischen Parametern durchführt: 1 V bis 160 V, 1 nC bis 1µC, Impulse von 1 Hz bis 100 Hz. Es werden also kurze Pulse mit einer elektrischen Spannung zwischen 1 V und 160 V auf die Haut gelenkt. Dabei wird innerhalb eines Pulses eine Ladung zwischen 1 nC bis zu einem µC auf der Haut entladen und dies mit einer Frequenz zwischen 1 Hz und 100 Hz. Es versteht sich von selbst, dass die elektrische Stimulation der Haut so durchgeführt wird, dass der Nutzer keinen elektrischen Schlag bekommt. Bei einer Stimulation mit 160 V-Impulsen ist die Ladung so gering, beispielsweise mehrere nC, dass trotz der hohen Spannung kein elektrischer Schlag zu fühlen ist. Bei geringeren Spannungen im einstelligen Volt-Bereich kann die Ladung schon erheblich größer sein. Allgemein gilt, dass entweder eine hohe Spannung oder ein größerer Strom pro Fläche als elektrische Reizung geeignet ist. Bei sehr kurzen Impulsen ist es nicht sinnvoll, von einem elektrischen Strom zu definieren, da sich der Querschnitt auf der Haut stark verbreitert und es durch die Elektroden unterschiedliche Kontakte gibt, die Punktkontakte aber auch Flächenkontakte sein können. Um die Wirkung der elektrischen Stimulation zu verbessern, hat es sich als vorteilhaft herausgestellt, wenn vorgesehen ist, dass die elektrischen Impulse der Steuerungsvorrichtung mit einer elektrischen Depolarisation der Haut beginnen und einer unmittelbar anschließenden Polarisation der Haut fortfahren. Durch die kurzzeitige Depolarisation mit nachfolgender Polarisation wird die Wirkung der der elektrischen Impulse auf die Stimulation der Haut verstärkt.

Die Erfindung wird anhand der folgenden Figuren näher erläutert. Es zeigt:
- Fig. 1: eine erste Variante der erfindungsgemäßen Vorrichtung zur elektrischen Stimulation der Kopfhaut in Form einer Kappe mit elektrischem Versorgungskabel,
- Fig. 2: eine zweite Variante der erfindungsgemäßen Vorrichtung zur elektrischen Stimulation der Kopfhaut in Form einer Kappe mit Akkumulator und einer Vorrichtung zur drahtlosen Datenübermittlung,
- Fig. 3: die Vorrichtung aus Figur 1 mit Einblick in das Kappenfutter,
- Fig. 4: die Vorrichtung aus Figur 2 mit Einblick in das Kappenfutter,
- Fig. 5: eine Stimulationselektrode der Vorrichtung in Figur 1,
- Fig. 6: eine Stimulationselektrode der Vorrichtung in Figur 2,
- Fig. 7: eine zweite Variante einer Stimulationselektrode mit Kontaktspitzen für die Vorrichtung nach Figur 1,
- Fig. 8: eine zweite Variante einer Stimulationselektrode mit Kontaktspitzen für die Vorrichtung nach Figur 2,

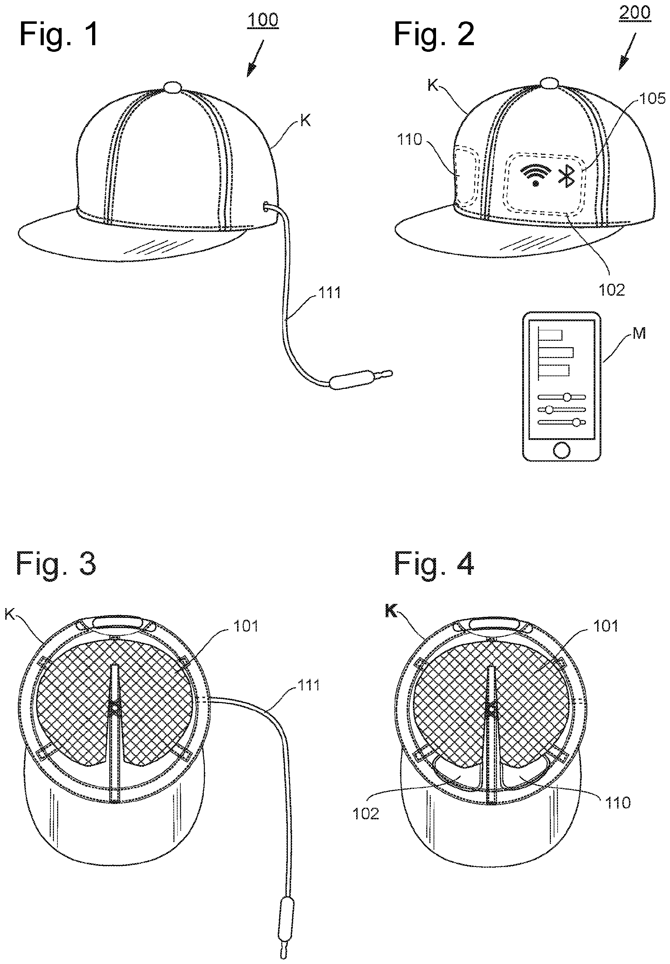

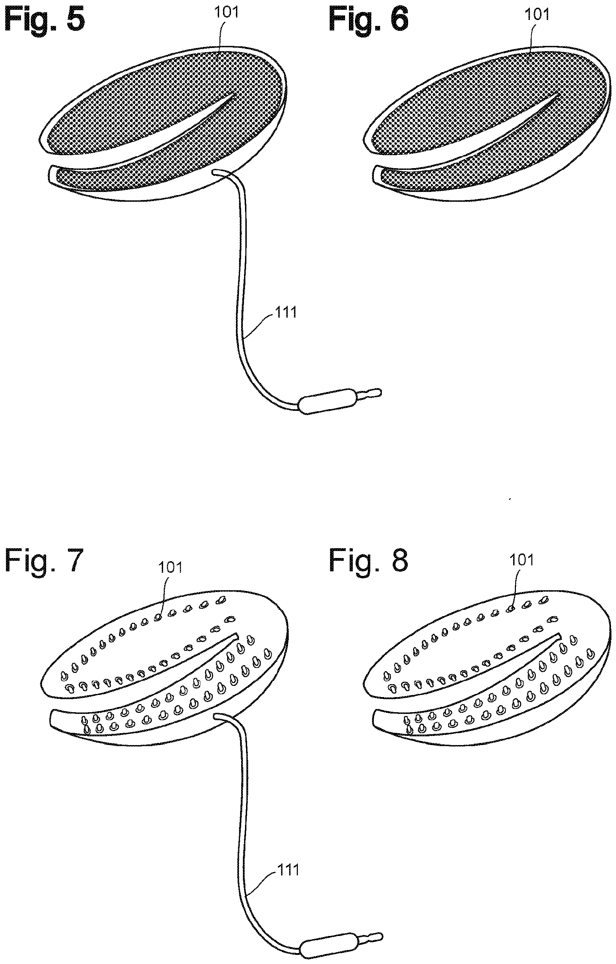

- Fig. 8: eine dritte Variante einer Stimulationselektrode mit Kontaktspitzen für die Vorrichtung nach Figur 1 oder Figur 2,

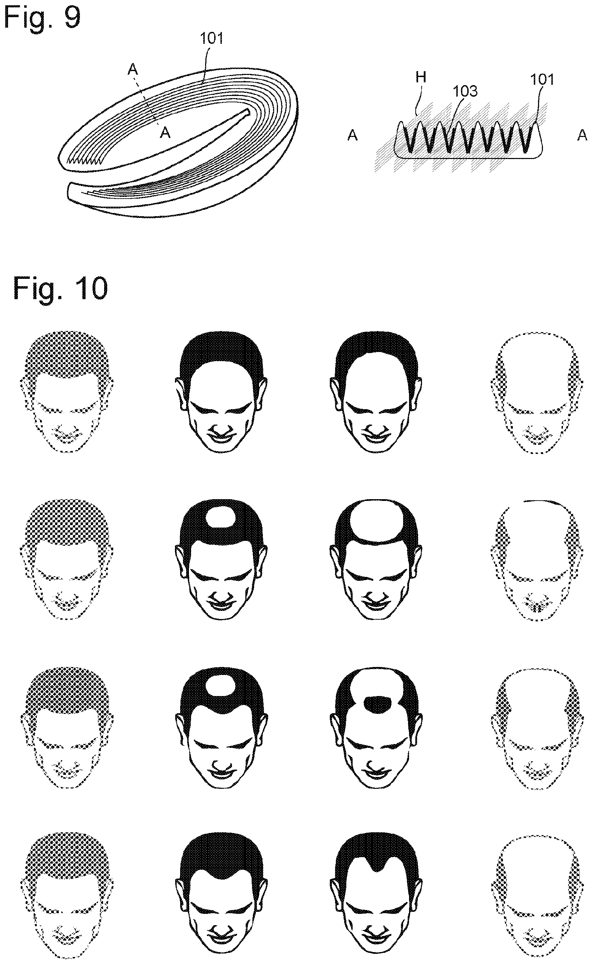

- Fig. 10: eine Darstellung von typischen Haarverluststadien eines Mannes,

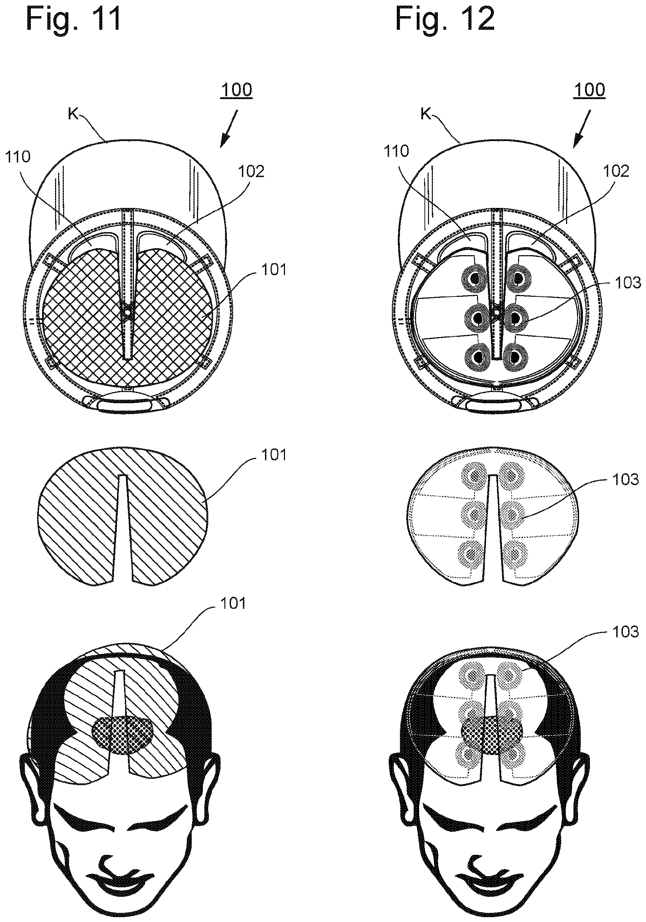

- Fig. 11: eine Skizze zur Verdeutlichung, wie die Stimulationselektrode in dem Kappenfutter liegt und mit der Oberfläche des Kopfes in Berührung kommt,
- Fig. 12: eine Skizze zur Verdeutlichung, wie die der Detektor in dem Kappenfutter liegt und mit der Oberfläche des Kopfes in Berührung kommt,

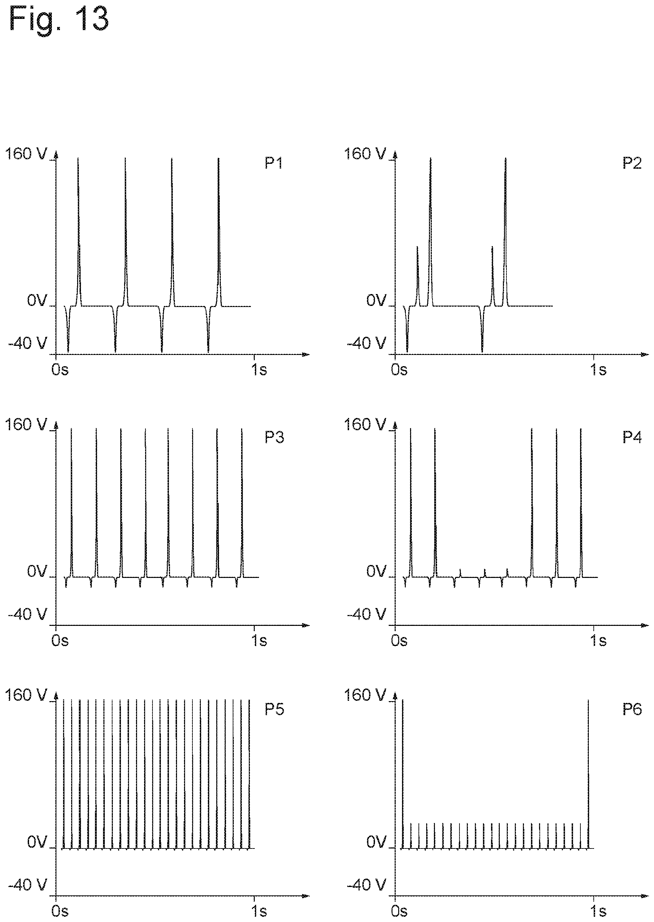

- Fig. 13: eine Zusammenstellung von beispielhaften elektrischen Stimulationsmustern,

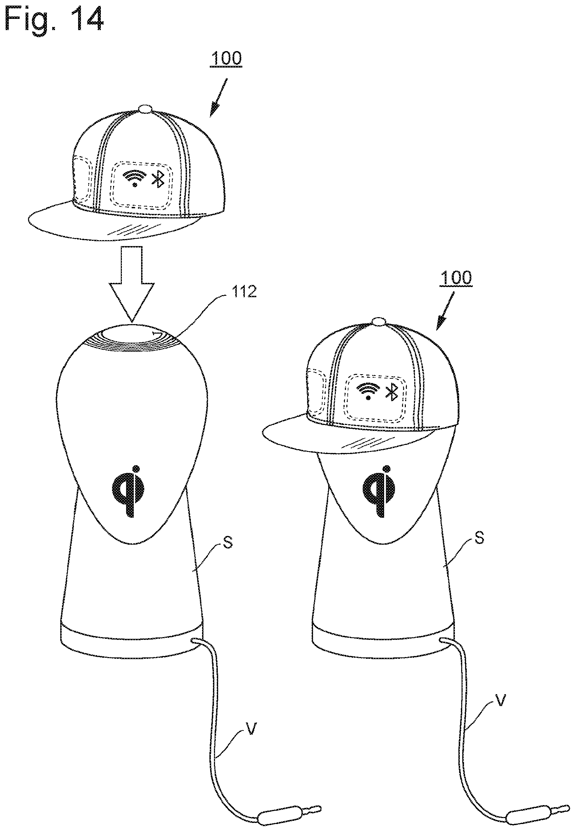

- Fig. 14: eine Skizze zur Verdeutlichung der Aufbewahrung der Vorrichtung auf einem Ständer mit kontaktloser Ladevorrichtung.

In **Figur 1** ist eine erste Variante der erfindungsgemäßen Vorrichtung 100 zur elektrischen Stimulation der Kopfhaut in Form einer Kappe K mit elektrischem Stromversorgungskabel 111 dargestellt. Das Stromversorgungskabel 111 kann mit einer elektrischen Stromversorgung verbunden werden, die am Körper, beispielsweise in einer Jackeninnentasche getragen wird. Dabei besteht die elektrische Versorgungeinrichtung aus einem am Markt verfügbaren, wiederaufladbaren elektrischen Batterie, wie diese als "Powerbanks" für Mobiltelefone erhältlich sind. Diese Vorrichtung ist geeignet um Langzeitstimulationsprogramme mit relativ hohem Stromverbrauch abzudecken.

In **Figur 2** hingegen, ist eine zweite Variante 200 der erfindungsgemäßen Vorrichtung zur elektrischen Stimulation der Kopfhaut in Form einer Kappe K mit Ackumulator 110 und einer Vorrichtung 105 zur drahtlosen Datenübermittlung dargestellt. Integriert mit der Vorrichtung 105 zur drahtlosen Datenübermittlung ist eine Steuerungseinrichtung 102, welche zur Stimulation der Kopfhaut verschiedene Programme durchführen kann, wobei die Steuerungsvorrichtung 102 zur Auswahl der Programme auf Daten zurückgreifen kann, die von mindestens einer Detektorvorrichtung 103 zur Detektion des Haarversluststatus stammt. Daten aus der Steuerungsvorrichtung 102 werden an die Vorrichtung 105 zur drahtlosen Datenübermittlung gegeben und können vor dort aus an ein Mobiltelefon M zur Auswertung geleitet werden. Auch ist es möglich, mit dem Mobiltelefon M Funktionsparameter die Steuerungsvorrichtung 102 einzustellen.

In **Figur 3** ist die Vorrichtung aus Figur 1 mit Einblick in das Kappenfutter als Skizze gezeigt. In dieser Unteransicht ist eine Elektrode 101 sichtbar, die hier als Folienelektrode ausgeführt ist und etwa den gesamten Bereich des Kappenfutters einnimmt. Dabei befinden sich auf der Folienelektrode hier nicht eingezeichnete Spikes mit runder Spitze, die noch vorhandenes Haar durchdringen, und mit ihrer runden Spitze auf der Kopfhaut aufliegen. Dabei durchdringen die Spikes die Kopfhaut nicht, so dass eine nichtinvasive Elektrode mit einer Vielzahl von Kontaktpunkten vorliegt.

In **Figur 4** ist die Vorrichtung aus Figur 2 mit Einblick in das Kappenfutter als Skizze gezeigt. In dieser Unteransicht ist ebenfalls eine Elektrode 101 sichtbar, die auch hier als Folienelektrode mit Spikes mit runden Spitzen als Kontaktpunkt zur Übertragung der elektrischen Reize auf die Kopfhaut ausgeführt ist und etwa den gesamten Bereich des Kappenfutters einnimmt. In dieser Darstellung sind auch der Akkumulator 110 und die integrierte Steuerungsvorrichtung 102 etwa im Stirnbereich der Kappe sichtbar.

Die Stimulationselektrode 101 der Vorrichtung in Figur 1 ist in **Figur 5** vereinzelt dargestellt. Deutlich ist zu sehen, dass die Folienelektrode mit Spikes mit runden Spitzen in dieser Ausgestaltung konkav ausgestaltet ist, um sich an die etwa ellipsoide Kopfform anzupassen. Durch die konkave Form ist der Abstand der als Folienelektrode ausgestaltete Elektrode 101 zur lokalen Oberfläche des Kopfes stets etwa gleich groß. In dieser Ausgestaltung der Vorrichtung zur Stimulation des Haarwuchses ist die Elektrode 101 unmittelbar mit einem elektrischen Stromversorgungskabel 111 verbunden. In **Figur 6** ist jedoch das Pendant ohne externes Stromversorgungskabel gezeigt. Diese Elektrode ist in Figur 2 mit dem Akkumulator 110 und der Steuerungsvorrichtung 102 verbunden.

Eine Alternative zu den als Folienelektrodenmit Spikes mit runden Spitzen ausgestalteten Elektroden 101 in den Figuren Figur 5 und Figur 6 ist in den Figuren **Figur 7** und **Figur 8** gezeigt. Diese Elektroden 101 sind als vereinzelte Stifte ausgeführt und reichen durch das Kopfhaar bis unmittelbar an die Kopfhaut heran und erzeugen so einen unmittelbaren Kontakt mit der Kopfoberfläche. Auch diese Stifte durchdringen die Kopfhaut nicht und bilden eine nichtinvasive Kopfhautelektrode. Durch den dadurch verringerten Übergangswiderstand ist es möglich, mit Stimulationsimpulsen mit geringerer elektrischer Spannung zu arbeiten. In Figur 7 sind die einzelnen als Stiftelektroden ausgeführten Elektroden 101 mit dem Stromversorgungskabel 111 verbunden. In der rechten Darstellung in Figur 8 sind die gleichen Elektroden 101 dargestellt, die nicht mit einem Stromversorgungskabel 111 verbunden sind, sondern diese Elektroden 101 sind mit dem Ackumulator 110 und der Steuerungsvorrichtung 102 in Figur 2 verbunden.

In **Figur 9** ist eine dritte Variante der Stimulationselektrode 101 in Form einer sehr besonderen Elektrodenanordnung dargestellt. Die Elektroden 101 sind in berg- und talform angeordnet. Auf den Abhängen der Elektroden 101 befinden sich Detektorvorrichtungen 103, welche die Dichte des zwischen ihnen befindliche Haares H über Impedanzmessungen ermittelt. Die Elektroden 101 werden von der Steuerungseinrichtung 102 in Abhängigkeit von der gemessenen Haardichte variiert. Die Anordnung der Elektroden 101 kann, wie in Figur 9 dargestellt, als umlaufende Elektrode ausgeführt sein. Es ist aber auch möglich, die Detektorvorrichtungen 103 an den Seiten der Stifte der Elektroden 101, die in den Figuren 5 und 6 gezeigt sind, anzubringen.

In **Figur 10** ist eine Matrix von typischen Haarversluststadien gezeigt. Je nach physischer Konstitution der betroffenen Person durchläuft der Haarverlust die Stadien von links nach rechts. Beginnend links bei vollem Haar (schattiert) durchläuft eine von Haarverlust betroffene Person eine stets größer werdende Stirn (1. reihe), oder erfährt einen kreisrunden Haarausfall (2. Reihe), wobei sich der Kreis stets vergrößert. Dabei unterscheidet sich das fortschreitende Stadium des Haarverlustes bei einigen dadurch, dass im Bereich der oberen Stirn ein kreisrundes Büschel übrigbleibt (3. Reihe). Eine weitere Variante der Haarverluststadien ist in der 4. Reihe gezeigt. Bei diesen Stadien wachsen die sogenannten "Geheimratsecken" bis auch hier die Glatze entsteht, die in allen Verläufen zu einer Art Tonsur auswächst. Diese unterschiedlichen Haarverluststadien festzustellen und die Stimulation nur in den betroffenen Kopfhautbereichen durchzuführen, ist die Wirkung der hier vorgestellten Vorrichtung zur Stimulation des Haarwuchses.

In den Figuren **Figur 11** und **Figur 12** ist gezeigt, wie die Elektrode 101 (Figur 11) und die Detektorvorrichtung 103 in Schichten unmittelbar aufeinander liegen. Von oben zeigen die einzelnen Skizzen in Figur 11 den Blick in das Futter der Kappe mit sichtbarer Folienelektrode mit Spikes mit runden Spitzen als Kontaktpunkt (vgl. Figur 4). Die Kappe kippt in dem mittleren Bild mit der Sonnenblende aus der Papierebene heraus nach vorne. Um die Folienelektrode in dieser Stellung zu zeigen, wurde in der mittleren Darstellung die Kappe ausgeblendet, so dass die Folienelektrode von der Seite zu sehen ist, die am Futter der Kappe K liegt. In dieser Stellung legt sich die konkave Folienelektrode (vgl. Figur 5 und Figur 6) oder die konkave Anordnung einzelner Elektroden (vgl. Figur 7 und Figur 8) auf die betroffene Oberfläche des Kopfes. In Figur 12 ist die Detektoranordnung 103 gezeigt, die unmittelbar mit der Folienelektrode oder mit der Anordnung einzelner Elektroden verbunden ist. Die Detektorvorrichtung 103 weist mehrere Flachspulen auf, mit deren Hilfe über eine lokale Impedanzmessung der Status des Haarverlustes an typischen Stellen des Kopfes detektiert werden kann. In dieser Ausgestaltung befinden sich die Detektoren links und rechts der Fontanelle. Es ist aber auch möglich, die Elektroden anders zu verteilen, um die typischen Stadien des Haarverlustes detektieren zu können.

In **Figur 13** sind verschiedene elektrische Stimulationsmuster als beispielhafte Programme P1, P2. P3, P4, P5 und P6 gezeigt. Es ist möglich, beispielsweise die Kopfhaut mit kurzen Impulsen mit einer Frequenz von 1Hz bis 4Hz zu stimulieren. Um den Effekt der Stimulation zu erhöhen, kann vorgesehen sein, zunächst eine Depolarisation vorzunehmen, wie in den beispielhaften Programmen P1 und P2. Auf die Depolarisation folgt eine Polarisation mit einem einmal-Impuls wie in Programm P1 oder eine doppelte Pulsfolge wie in Programm P2. Das beispielhafte Programm P3 besteht aus einer etwas schnelleren Pulsfolge mit gleichmäßigen Pulsen ohne eine vorherige Depolarisation. Es ist auch möglich, dass die Impulse in Pulsgruppen geschehen, wie in dem beispielhaften Programm P4. In dem beispielhaften Programm P5 ist eine Pulsfolge mit etwa 25 Hz gezeigt. Noch eine andere Pulsfolge ist in Programm P6 mit Niedervoltimpulsen gezeigt, in die mit einer Frequenz von etwa 1 Hz Impulse mit höher Spannung eingesetzt sind. Insgesamt kann die Frequenz der Impulse zwischen 1 Hz und 100 Hz betragen und dabei unterschiedliche Depolarisations- / Polarisationsmuster zeigen.

In **Figur 14** ist schließlich ein Ständer für die Vorrichtung zur Stimulation des Kopfhaarwuchses 100 dargestellt, der an einen Perückenständer erinnert. In die obere Oberfläche des Ständers S ist eine Spule 112 eingelegt, die mit einem Stromversorgungskabel V verbunden ist. Über diese Spule 112 lässt sich der Ackumulator 110 der Vorrichtung zur Stimulation des Kopfhaarwuchses drahtlos laden. Dabei kann als Ladeprotokoll das Qi-Protokoll verwendet werden, das auch bei Mobiltelefonen eingesetzt wird.

Die hier vorgestellte Erfindung ist geeignet zur nichtinvasiven Elektrostimulation des Kopfhaarwuchses, wenn es sich um eine Ausführungsform in Form eines Hutes oder einer Kappe handelt, und auch der nichtinvasiven Elektrostimulation der Behaarung anderer Körperteile, wie zum Beispiel die Behaarung der Brust oder des Rückens, die Behaarung der Achseln aber auch die Behaarung der Scham oder zur Stimulation des Bartwuchses, wenn es sich um eine Ausführungsform in Form eines anderen Bekleidungsstückes handelt. Hierzu ist es möglich, dass die Vorrichtung als tragbare Vorrichtung in Form eines Kleidungsstücks als sogenanntes "Wearable" ausgestaltet ist, zum Beispiel in Form eine Oberbekleidungsstücks, wie ein Unterhemd, ein T-Shirt, ein Hemd, ein Pullover, eine Jacke, eine Unterhose, eine Sporthose, eine elastische Binde, wie zum Beispiels eine Binde zur Unterstützung der Rückenmuskulatur, oder als Schal oder als Bartbinde.

### BEZUGSZEICHENLISTE

| | | | |
|---|---|---|---|
| 100 | Vorrichtung zur Stimulation | K | Kappe |
| 101 | Elektrode | M | Mobiltelefon |
| 102 | Steuerungsvorrichtung | P1 | Programm |
| 103 | Detektorvorrichtung | P2 | Programm |
| 104 | Feuchtigkeitssensor | P3 | Programm |
| 105 | Vorrichtung zur drahtlosen Datenübertragung | P4 | Programm |
| | | P5 | Programm |
| 110 | Akkumulator | P6 | Programm |
| 111 | Stromversorgungskabel | S | Ständer |
| 112 | Spule | V | elektrisches Versorgungskabel |
| 200 | Vorrichtung zur Stimulation | | |

## Patentansprüche

1. Vorrichtung (100, 200) zur Stimulation des Haarwuchses, aufweisend
- mindestens eine Elektrode (101) zur elektrischen Reizung der Haut, wobei eine mit der mindestens einen Elektrode (101) verbundene Steuerungsvorrichtung (102) elektrische Impulse auslöst,
**dadurch gekennzeichnet, dass**
die Steuerungsvorrichtung (102) mit mindestens einer Detektorvorrichtung (103) verbunden ist, über welche das Maß des vorliegenden Haarverluststatus oder der Behaarung detektierbar ist, wobei die Steuerungsvorrichtung (102)
- in Abhängigkeit von dem detektierten Haarstatusverlustes oder der detektierten Behaarung die elektrische Reizung kontinuierlich variiert und/oder
- in Abhängigkeit von dem detektierten Haarstatusverlust ein voreingestelltes Stimulationsprogramm (P1, P2, P3, P4, P5, P6) zur elektrischen Stimulation der Haut ausführt,
wobei die mindestens eine Elektrode (101) mit einer Vielzahl von Spikes mit runden Spitzen, Stiften oder in Berg-und Talform ausgestattet ist, welche durch noch vorhandenes Haar ragen und die Haut nicht durchdringen, sondern auf der Haut aufliegen, wobei
die Steuerungsvorrichtung (102) des Weiteren mit einem Feuchtigkeitssensor (104) verbunden ist, über welche die elektrische Leitfähigkeit der Kopfhaut ableitbar ist, wobei die Steuerungsvorrichtung (102) in Abhängigkeit von der detektierten Feuchtigkeit das voreingestelltes Stimulationsprogramm (P1, P2, P3, P4, P5, P6) variiert.

2. Vorrichtung nach Anspruch 1,
wobei die Steuerungsvorrichtung (102) mit einer Mehrzahl von Detektorvorrichtungen (103) verbunden ist, wobei jede einzelne Detektorvorrichtung (103) einen vorbestimmten Bereich der Haut abdeckt.

3. Vorrichtung nach einem der Ansprüche 1 oder 2,
wobei die Steuerungsvorrichtung (102) des Weiteren mit einer Vorrichtung (105) zur drahtlosen Datenübertragung verbunden ist, wie beispielsweise ein BlueTooth^{®}-Modul oder ein WLAN-Modul, über welche der Status der aktuelle Steuerungsvorrichtung (102) auslesebar ist.

4. Vorrichtung nach Anspruch 1 bis 3,
wobei die Steuerungsvorrichtung (102) des Weiteren mit einer Vorrichtung (105) zur drahtlosen Übertragung verbunden ist, wie beispielsweise ein Blue-Tooth^{®}-Modul oder ein WLAN-Modul, über welche der Status der Steuerungsvorrichtung (102) veränderbar ist.

5. Vorrichtung nach Anspruch 1 bis 4,
wobei die Vorrichtung (100, 200) in Form eines Hutes oder einer Kappe aufgebaut ist, wobei die mindestens eine Elektrode (101) und die mindestens eine Detektorvorrichtung (103) innerhalb des Hutes oder innerhalb der Kappe angeordnet sind.

6. Vorrichtung nach Anspruch 5,
wobei dass innerhalb des Hutes oder innerhalb der Kappe ein Akkumulator (110) zur Stromversorgung angeordnet ist.

7. Vorrichtung nach einem der Ansprüche 1 bis 6,
wobei der Hut oder die Kappe zum Laden des Akkumulators (110) ein Stromversorgungskabel (111) aufweist, oder dass
im Hut oder in der Kappe eine Spule (112) zur induktiven Ladung des Ackumulators (110) vorhanden ist.

8. Vorrichtung nach einem der Ansprüche 1 bis 7,
wobei die Steuerungsvorrichtung (102) die Reizung der Haut mit folgenden elektrischen Parametern durchführt: 1 V bis 160 V, 1 nC bis 1µC, Impulse von 1Hz bis 100 Hz.

9. Vorrichtung nach Anspruch 8,
wobei die elektrischen Impulse der Steuerungsvorrichtung (102) mit einer elektrischen Depolarisation der Haut beginnen und einer unmittelbar anschließenden Polarisation der Haut fortfahren.

## Claims

1. A device (100, 200) for stimulating hair growth, having
- at least one electrode (101) for electrically stimulating the skin, a control device (102), which is connected to the at least one electrode (101), triggering electrical pulses,
**characterized in that**
the control device (102) is connected to at least one detector device (103), by means of which the extent of the present hair loss status or amount of hair can be detected, wherein the control device (102)
- depending on the detected hair status loss or the detected amount of hair, continuously varies the electrical stimulation, and/or
- depending on the detected hair status loss, executes a preset stimulation program (P1, P2, P3, P4, P5, P6) for electrically stimulating the skin,
wherein the at least one electrode (101) is equipped with a multiplicity of spikes with round tips, pins or in a hill and valley shape, which protrude through hair that is still present and do not penetrate the skin, but rather rest on the skin, wherein
the control device (102) is further connected to a moisture sensor (104), by means of which the electrical conductivity of the scalp can be derived, wherein the control device (102) varies the preset stimulation program (P1, P2, P3, P4, P5, P6) depending on the detected moisture.

2. The device according to Claim 1,
wherein
the control device (102) is connected to a plurality of detector devices (103), wherein each individual detector device (103) covers a predetermined region of the skin.

3. The device according to either of Claims 1 and 2,
wherein
the control device (102) is further connected to a device (105) for wireless data transmission, such as for example a Bluetooth^{®} module or a WLAN module, by means of which the status of the current control device (102) can be read.

4. The device according to Claims 1 to 3,
wherein
the control device (102) is further connected to a device (105) for wireless transmission, such as for example a Bluetooth^{®} module or a WLAN module, by means of which the status of the control device (102) can be changed.

5. The device according to Claims 1 to 4,
wherein
the device (100, 200) is structured in the form of a hat or a cap, wherein the at least one electrode (101) and the at least one detector device (103) are arranged inside the hat or inside the cap.

6. The device according to Claim 5,
wherein
a rechargeable battery (110) for supplying power is arranged inside the hat or inside the cap.

7. The device according to one of Claims 1 to 6,
wherein
the hat or the cap has a power supply cable (111) for charging the rechargeable battery (110), or
a coil (112) for inductively charging the rechargeable battery (110) is present in the hat or in the cap.

8. The device according to one of Claims 1 to 7,
wherein
the control device (102) carries out the stimulation of the skin using the following electrical parameters: 1 V to 160 V, 1 nC to 1 µC, pulses from 1 Hz to 100 Hz.

9. The device according to Claim 8,
wherein
the electrical pulses of the control device (102) begin with an electrical depolarization of the skin and continue with a directly following polarization of the skin.

## Revendications

1. Dispositif (100, 200) pour stimuler la pousse des cheveux, comportant
- au moins une électrode (101) pour la stimulation électrique de la peau, sachant qu'un dispositif de commande (102) relié à au moins une électrode (101) déclenche des impulsions électriques,
**caractérisé en ce que**
le dispositif de commande (102) est relié à au moins un dispositif de détection (103) par le biais duquel la mesure de l'état de perte de cheveux présent ou de la chevelure peut être détecté, sachant que le dispositif de commande (102)
- varie la stimulation électrique en continu en fonction de la perte d'état de la chevelure détectée ou de la chevelure détectée, et/ou
- exécute un programme de stimulation préréglé (P1, P2, P3 , P4, P5, P6) pour la stimulation électrique de la peau en fonction de la perte d'état de la chevelure détectée,
sachant qu'au moins une électrode (101) est dotée d'une pluralité de pointes avec des bouts arrondis, de tiges ou en forme de montagnes russes, lesquelles dépassent à travers les cheveux encore existants et ne traversent pas la peau, mais reposent sur la peau, sachant que
le dispositif de commande (102) est relié en outre à un capteur d'humidité (104) par le biais duquel la conductibilité électrique de la peau de la tête peut être dérivée, sachant que le dispositif de commande (102) varie le programme de stimulation préréglé (P1, P2, P3 , P4, P5, P6) en fonction de l'humidité détectée.

2. Dispositif selon la revendication 1,
sachant que
le dispositif de commande (102) est relié à une pluralité de dispositifs de détection (103), sachant que chaque dispositif de détection (103) individuel couvre une zone prédéterminée de la peau.

3. Dispositif selon l'une quelconque des revendications 1 ou 2,
sachant que
le dispositif de commande (102) est relié en outre à un dispositif (105) pour la transmission de données sans fil, comme par exemple un module Blue-Tooth^{®} ou un module de réseau local sans fil (WLAN) par le biais duquel l'état du dispositif de commande actuel (102) peut être lu.

4. Dispositif selon la revendication 1 à 3,
sachant que
le dispositif de commande (102) est relié en outre à un dispositif (105) pour la transmission sans fil, comme par exemple un module Blue-Tooth^{®} ou un module de réseau local sans fil (WLAN) par le biais duquel l'état du dispositif de commande (102) peut être modifié.

5. Dispositif selon l'une quelconque des revendications 1 à 4,
sachant que
le dispositif (100, 200) est constitué sous la forme d'un chapeau ou d'une casquette, sachant qu'au moins une électrode (101) et au moins un dispositif de détection (103) sont disposés à l'intérieur du chapeau ou à l'intérieur de la casquette.

6. Dispositif selon la revendication 5,
sachant qu'
une batterie (110) est disposée à l'intérieur du chapeau ou à l'intérieur de la casquette pour l'alimentation en courant.

7. Dispositif selon l'une quelconque des revendications 1 à 6,
sachant que
le chapeau ou la casquette comporte un câble d'alimentation en courant (111) pour charger la batterie (110) ou qu'une bobine (112) est présente dans le chapeau ou dans la casquette pour la charge inductive de la batterie (110).

8. Dispositif selon l'une quelconque des revendications 1 à 7,
sachant que
le dispositif de commande (102) exécute la stimulation de la peau avec les paramètres électriques suivants : 1 V à 160 V, 1 nC à 1 µC, impulsions de 1 Hz à 100 Hz.

9. Dispositif selon la revendication 8,
sachant que
les impulsions électriques du dispositif de commande (102) commencent avec une dépolarisation électrique de la peau et continuent aussitôt après avec une polarisation de la peau.
